(19) Europäisches Patentamt
European Patent Office
Office européen des brevets

(11) Veröffentlichungsnummer : **0 071 704**
**B1**

(12) # EUROPÄISCHE PATENTSCHRIFT

(45) Veröffentlichungstag der Patentschrift : **30.12.86**

(21) Anmeldenummer : **82104288.4**

(22) Anmeldetag : **15.05.82**

(51) Int. Cl.⁴ : **C 08 J 9/26**, C 08 J 9/28, C 12 N 11/00, B 01 J 20/00, C 07 K 17/00, A 61 K 37/48, A 61 K 47/00// C08F2/22

(54) **Oberflächenreiche Systeme zur Fixierung von nucleophile Gruppen enthaltenden Substraten.**

(30) Priorität : **05.08.81 DE 3130924**

(43) Veröffentlichungstag der Anmeldung : **16.02.83 Patentblatt 83/07**

(45) Bekanntmachung des Hinweises auf die Patenterteilung : **30.12.86 Patentblatt 86/52**

(84) Benannte Vertragsstaaten : **AT CH FR GB IT LI NL SE**

(56) Entgegenhaltungen :
**WO-A-81 /014 12**
**DE-A- 2 016 729**
**DE-A- 2 639 234**
**DE-A- 2 815 908**
**DE-A- 2 941 881**
**Die Akte enthält technische Angaben, die nach dem Eingang der Anmeldung eingereicht wurden und die nicht in dieser Patentschrift enthalten sind.**

(73) Patentinhaber : **Röhm GmbH**
**Kirschenallee Postfach 4242**
**D-6100 Darmstadt 1 (DE)**

(72) Erfinder : **Siol, Werner, Dr.**
**Mühlbergstrasse 4**
**D-6102 Pfungstadt (DE)**
Erfinder : **Krämer, Dieter, Dr.**
**An der Favorite 4**
**D-6500 Mainz (DE)**
Erfinder : **Sütterlin, Norbert, Dr.**
**Am Horeth 23**
**D-6105 Ober-Ramstadt (DE)**
Erfinder : **Fell, Cornelia**
**Rheinstrasse 36**
**D-6106 Erzhausen (DE)**
Erfinder : **Markert, Gerhard, Dr.**
**Leipziger Strasse 25**
**D-6105 Ober-Ramstadt (DE)**
Erfinder : **Schuster, Erwin, Dr.**
**Darmstädter Strasse 237**
**D-6140 Bensheim 3 (DE)**

**EP 0 071 704 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft oberflächenreiche Systeme zur Fixierung von nucleophile Gruppen enthaltenden Substraten, insbesondere zur Immobilisierung von biologisch relevanten, d. h. primär zur Wechselwirkung mit biologischen Systemen befähigten Substanzen und funktionalen Einheiten, vorzugsweise biologischen Ursprungs.

Die Immobilisierung von Bio(makro)molekülen an Trägermolekülen ist eines der am intensivsten bearbeiteten Themen sowohl im Bereich der reinen Forschung als auch im Bereich der Biotechnologie.

Unter dem speziellen Aspekt der Immobilisierung von Enzymen kann man die vorgeschlagenen bzw. praktizierten Fixierungstechniken wie folgt unterscheiden :

1) Kovalente Bindung an eine feste Trägerphase (solid phase)
2) Kovalente Bindung an lösliche Polymere
3) Physikalische Adsorption an eine feste Trägerphase
4) Vernetzung an festen Oberflächen
5) Vernetzung mit bifunktionellen Reagentien
6) Einschluß in einer Gelphase
7) Einkapselung

(Vgl. R. D. Fall in « Enzyme Engineering » Vol. II, ed. E. K. Pyl & L. B. Wingard, Plenum Press. 1974, US-PS 3 650 900 Melrose, Rev. Pure and Appl. Chem. *21*, 83-119 (1971).

Am eingehendsten wurde bisher die unter 1) genannte Technik der kovalenten Bindung an eine feste Trägerphase bearbeitet.

Die einschlägige Literatur läßt jedoch keinen Zweifel an der Tatsache, daß die vielfältigen Aufgaben, die man mit Hilfe der Fixierung von Bio-Makromolekülen zu lösen hoffte, wie z. B. Reinigung, Trennung und Bindung von Enzymen, Fixierung von Mikroorganismen, Affinitätschromatographie, Immunreaktionen, Aufgaben der klinischen Diagnostik usw. nicht mit einer einzigen Methodik gelöst werden können. Auch in Fällen, wo auf spezifische Probleme zugeschnittene Lösungen vorliegen, z. B. bei der Immobilisierung bestimmter Enzyme an bestimmten Trägern, stößt die Übertragung vom Labormaßstab in den technischen Maßstab oft auf schwer zu überwindende Hindernisse.

Es hat daher nicht an Versuchen gefehlt, zu Lösungen zu kommen, die die Bedürfnisse der Technik besser befriedigen.

Die japanische Offenlegungsschrift 77 143 821 beschreibt die Immobilisierung von Enzymen oder Mikroben durch ein Verfahren, in dem aus einer wäßrigen Polymerdispersion und einem Enzym auf einer Glasplatte ein Film hergestellt wird. Die Anwendung geschieht in Form einer, gegebenenfalls zerkleinerten Folie.

Die Lösungen des Standes der Technik weisen schwerwiegende Nachteile auf. In der Regel ist die Oberflächenkonzentration der fixierten, biologisch wirksamen Substanzen zu gering. Die reaktiven Oberflächen können auch nicht beliebig durch Zerkleinern der polymeren Träger erhöht werden, da kleine Teilchen die Tendenz zur Instabilität besitzen und vielfach nicht mehr technisch sinnvoll einzusetzen sind. Zwar ist es bekannt (siehe oben), makromolekulare Verbindungen durch Adsorption an Träger zu binden, jedoch ist deren Verwendbarkeit beschränkt, da sie sich leicht wieder eluieren lassen.

In der DE-OS 21 12 740 wird ein Durchflußreaktor beschrieben, der einen makroporigen Reaktionskern, dessen polymere Oberfläche adsorptionsfördernde Nitril-, Säureamid- bzw. Ureidgruppen aufweist.

Nach erfolgter physikalischer Adsorption der Enzyme an der festen Trägerphase wird eine Vernetzung z. B. mittels eines Dialdehyds vorgenommen.

Aus der DE-OS 22 60 184 ist ein Verfahren zur Herstellung von trägerfixierten makromolekularen Verbindungen bekannt, bei dem eine makromolekulare Verbindung A zuerst mit einer Verbindung B umgesetzt wird, die wenigstens eine zur Kupplung mit der makromolekularen Verbindung A befähigte Funktion und wenigstens eine weitere zur Polymerisation befähigte Funktion aufweist, dann ein Molekularsiebmaterial eines die makromolekulare Verbindung A ausschließenden Vernetzungsgrads in entquollenem Zustand zugesetzt und die polymerisationsfähige Gruppe des Kupplungsprodukts AB im Molekularsiebmaterial gegebenenfalls zusammen mit weiteren Monomeren polymerisiert wird.

Die DE-A-20 16 729 sieht ein Verfahren zur Herstellung unlöslicher Enzyme in aktiver Form vor, bei dem ein Mischpolymerisat mit einer Enzymlösung behandelt und eine Quervernetzung des Enzym-Polymerkomplexes und/oder eine Neutralisation der restlichen reaktionsfähigen chemischen Gruppen durchgeführt wird. Die Lehre dieser Offenlegungsschrift umfaßt die Auflösung jeglicher Partikelstruktur — soweit von der Herstellung der vorhanden — durch Auflösung der Mischpolymerisate in Lösungsmitteln. Damit entfällt die Voraussetzung für das Vorhandensein einer Partikelstruktur bei der Verfilmung. Soweit unlösliche Niederschläge oder Präcipitate den Gegenstand der Lehre bilden, sind sie nicht das unmittelbare Ergebnis des Polymerisationsverfahrens, sondern Folgeprodukte einer (externen) Vernetzung des gebildeten polymeren Materials, welche die Zugänglichkeit eher herabsetzt.

Die besonders günstigen oberflächenreichen Systeme sind nach dem Verfahren dieser Offenlegungsschrift nicht zu erwarten.

Es wurde nun gefunden, daß oberflächenreiche Systeme mit reaktiven Einheiten zur Bindung von nucleophile Gruppen enthaltenden Substraten besonders vorteilhafte Lösungen darstellen, wenn die reaktiven Einheiten zur Bindung der die nucleophilen Gruppen enthaltenden Substrate von der Herstellung her Bestandteile eines durch Emulsionspolymerisation hergestellten Polymerlatex sind, der aus 0,03 bis 6 µm großen Latexteilchen aufgebaut ist, und der Polymerlatex selbst zu einem oberflächenreichen System aggregiert und/oder an einem oberflächenreichen Trägermaterial fixiert ist.

Es wurde weiter gefunden, daß sich zur kovalenten Immobilisierung von biologisch relevanten Substanzen und funktionalen Einheiten Polymerlatices gemäß den Patentansprüchen besonders eignen.

Der Polymerlatex

Bei der Synthese der erfindungsgemäßen Polymerlatices ist von bestimmendem Einfluß die beabsichtigte Verwendung bei der Herstellung reaktiver oberflächenreicher Gebilde. Der Polymerlatex kann daher von unterschiedlichem Aufbau sein, abhängig davon, ob der Latex zur Erzeugung eines dünnen, reaktiven Films auf einem an sich oberflächenreichen Gebilde verwendet werden soll, oder ob der Latex durch einen locker agglomerierten Aufbau die Gesamtoberfläche des gebildeten Systems gegenüber der Oberfläche des Trägermaterials noch erheblich vergrößern soll. Falls die Erzeugung eines dünnen, reaktiven Films auf dem Trägermaterial angestrebt wird, kann der Latex bei einer Temperatur oberhalb der minimalen Filmbildungstemperatur (MFT = DIN 53787) auf den Träger aufgebracht werden. Soll der Latex hingegen die Oberfläche des Trägermaterials noch vergrößern, so sind insbesondere kleine, (beispielsweise im Bereich von 0,03 bis 3 µm) in sich starre Latex-Teilchen, die unter den Anwendungsbedingungen nicht verfilmen, von Vorteil. Gegebenenfalls können die nicht-filmbildenden Latexteilchen durch Zusatz von untergeordneten Mengen — vorzugsweise bis zu 30 Gew.-% — eines filmbildenden Latex miteinander und mit dem Träger verbunden werden.

Substrate

Die erfindungsgemäßen, oberflächenreichen Systeme sind allgemein zur Fixierung von Substraten geeignet, die nucleophile Gruppen aufweisen. Sie eignen sich besonders zur Fixierung von funktional und/oder morphologisch definierten, biologisch relevanten, insbesondere biologisch wirksamen Einheiten oder Substanzen.

Bei den biologisch relevanten Substanzen und funktionalen Einheiten, die in der Regel zur Wechselwirkung mit biologischen Systemen befähigt sind, handelt es sich vorzugsweise um solche biologischen Ursprungs, die gegebenenfalls gegenüber der nativen Form modifiziert sein können.

Eine besonders wichtige Rolle spielen dabei Makromoleküle, insbesondere Proteine.

Reaktive Einheiten/nucleophile Gruppen

Im allgemeinen besitzen die zur Wechselwirkung mit biologischen Systemen befähigten Substanzen bzw. Strukturen ihrerseits kopplungsfähige Gruppen, die mit den (reaktiven Einheiten der Polymerlatices) reagieren und eine kovalente Bindung eingehen können ; in der Regel sind dies nucleophile Gruppen. Vorzugsweise finden solche an sich bekannten reaktiven Einheiten (funktionellen Gruppen) Anwendung, die in wäßriger Lösung mit stärkeren Nucleophilen als Wasser reagieren und vom Wasser in dem physiologisch sinnvollen pH-Bereich, d. h. im Bereich von 5,0 bis 9,0, insbesondere von 6,5 bis 8,0 nicht oder nur in untergeordnetem Maße angegriffen werden. Die Auswahl der funktionellen Gruppen trägt der Tatsache Rechnung, daß das zu fixierende Material, insbesondere das Material biologischen Ursprungs als nucleophile Gruppe im allgemeinen die (freie) Aminogruppe, daneben gegebenenfalls noch phenolische-, Hydroxy- oder Thiolgruppen aufweist. Die erfindungsgemäßen Polymerlatices können ganz allgemein aus radikalisch polymerisierbaren Vinylverbindungen hergestellt werden :

Vorzugsweise aus Monomeren auf der Basis von Acrylsäure- und/oder Methacrylsäurederivaten und/oder Styrol und/oder Vinylestern, insbesondere Vinylacetat.

Der Aufbau des erfindungsgemäßen Polymerlatex in seiner reaktiven Form kann daher in stark schematisierter Form wie folgt dargestellt werden :

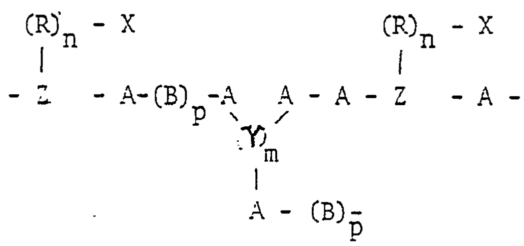

wobei X für die funktionellen Gruppen zur kovalenten Fixierung steht, vorzugsweise solchen, die die vorstehend genannten Bedingungen erfüllen. R stellt dabei einen « Abstandshalter » (Spacer) zwischen funktionellen und polymerisierbaren Einheiten dar. Größe und Typ des Abstandshalters sind vergleichsweise unkritisch. Typische Vertreter von derartigen Abstandshaltern sind beispielsweise Alkylengruppen von $C_1$ bis $C_{20}$, vorzugsweise $C_2$ bis $C_{12}$, wobei gegebenenfalls Kohlenstoffatome durch Ätherbrücken ersetzt sein können. Darüber hinaus andere ursprünglich (d. h. vor dem Einbau) bifunktionelle Gruppen enthaltende Einheiten, wobei sowohl am polymerständigen als am funktionalen Ende eine Verknüpfung über Amid-, Ester-, Äther-, Thioäther-, Harnstoff-, Urethan-, Sulfonamid- und ähnliche Gruppen erfolgen kann. Im allgemeinen bringt der Abstandshalter eine Distanz der funktionellen Gruppen X von der Polymerhauptkette im Bereich von 0,5-4 nm. In einer Reihe von Beispielen kann die Gruppe R ganz fehlen, d. h. n kann den Wert 0 oder 1 besitzen.

X hat in der Regel die Bedeutung einer von den in Frage kommenden Nucleophilen angreifbaren Gruppe, d. h. einer aktivierten Gruppe ; vorzugsweise die Bedeutung einer Sulfonsäurehalogenid-, einer Thioisocyanatgruppe, eines aktivierten Esters, einer Thiocarbonyldioxy-, Carbonyl-imidoyldioxy-, Haloethoxy-, Haloacetoxy-, Oxiran-, Aziridin-, Formyl-, Keto-, Acryloyl- oder Anhydridgruppe.

Als Sulfonsäurehalogenide kommen die Chloride und Bromide, als Haloacetoxy die Fluoro-, Chloro- und Bromoverbindungen, als Esterkomponente der aktivierten Ester solche von Hydroxylaminverbindungen, wie des N-Hydroxysuccinimids oder des N-Hydroxyphthalimids, von (mittels elektronenanziehenden Gruppen) aktivierten Phenolen, wie von Halogenphenolen, wie Trichlorphenol oder von Nitrophenolen, von heterocyclischen Lactamen, wie Pyridon infrage.

Besonders bevorzugt sind Oxiran-, Keto-, Formyl-, Sulfonsäurechlorid-, Thioisocyanatgruppen sowie aktivierte Carbonsäureester sowie Carbonsäureanhydride. Bei den Monomeren des Typs $Z'—(R)_n—X$ stellt demnach Z' eine (radikalisch) polymerisationsfähige Einheit und n 0 oder 1 dar.

Solche radikalisch polymerisationsfähige Einheiten sind z. B. Vinylgruppen, wobei Z' beispielsweise die Bedeutung

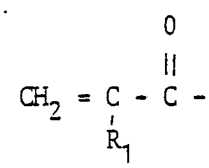

besitzt, worin $R_1$ für Wasserstoff oder Methyl bzw. für $CH_2—COOR_2$, $CH_2—CONHR_2$ oder $CH_2—CON(R_2)_2$ steht, wobei $R_2$ einen Alkylrest mit 1 bis 4 Kohlenstoffatomen bedeutet.

Desweiteren kann Z' sich von der Maleinsäure ableiten :

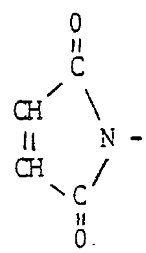

Als reaktionsfähige und zugleich polymerisierbare Einheiten sind ferner Maleinsäureanhydrid und Itaconsäureanhydrid anzusprechen sowie Acrolein, Methacrolein, Methylvinylketon und aktivierte Vinylester. Besonders bevorzugt sind Derivate der (Meth)-acrylsäure und des Maleinimids sowie Maleinsäure und Itaconsäureanhydrid.

Zur Verdeutlichung des Formelschemas $Z'—R—X$ seien die folgenden Beispiele aufgeführt :

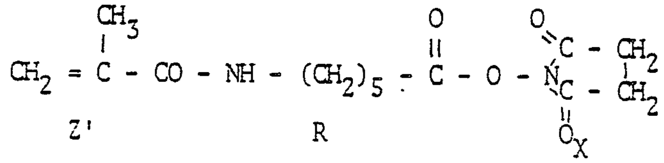

(Polymerisierbarer aktivierter Ester mit Spacer)

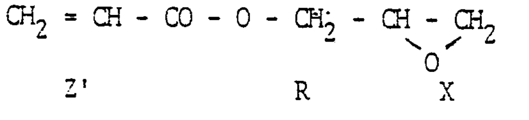 (Glycidylacrylat)

$$CH_2 = CCH_3 - CO - O - CH_2 - CH_2 - O - \underset{\underset{O}{\|}}{C} - CH_2 - Cl$$

Z' R X

(2-(Chloroacetoxy)-äthylmethacrylat)
$CH_2{=}CCH_3{-}CO{-}O{-}C_6H_2Cl_3$
(2,4,5-Trichlorphenylmethacrylat) R = 0
$CH_2{=}C(CH_3){-}COO{-}CH_2{-}CH_2{-}Br$
(2-Bromäthylmethacrylat)

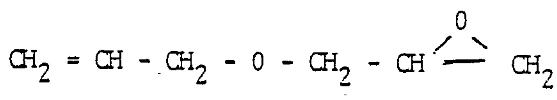

(Allylglycidyläther)

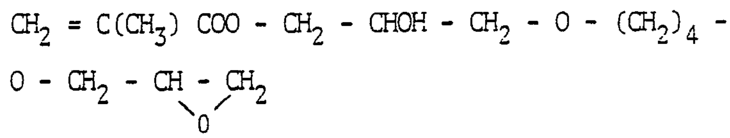

(Anlagerungsprodukt von Methacrylsäure an 1,4-Butandioldiglycidylether)
$CH_2{=}CH{-}COO{-}CH_2{-}CH_2{-}O{-}CSNH{-}(CH_2)_6{-}N{=}C{=}S$
(Anlagerungsprodukt von Acrylsäure-2-Hydroxyethylester an 1,6-Hexandiisothiocyanat)

$CH_2 = CH - O - CO - CH_2 - Cl$ (Chloressigsäurevinylester)

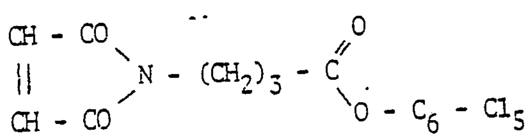

(4-Maleimido-buttersäure-pentachlorphenylester)
$CH_2{=}C(CH_3){-}COO{-}C_6H_4{-}SO_2{-}CH_3$
((4-Methylsulfinylphenyl)-methacrylat)
$CH_2{=}CH{-}COO{-}CH_2{-}C{\equiv}C{-}H$
(Propargylacrylat)

Bei den übrigen, am Aufbau der Polymerlatices beteiligten Einheiten (A und B in der schematischen Darstellung), handelt es sich definitionsgemäß um solche, die dem Latex die zu fordernden Eigenschaften, nämlich ggf. die Hydrophilie und die zweckmäßige Härte verleihen. Als Anhalt für die erwünschte Härte im wasserfreien Zustand kann gelten $T_{\lambda max}$ —60 bis 200 °C, insbesondere —20 bis 140 °C (nach DIN 53445). Andererseits sollten die am Aufbau des Polymerlatex beteiligten Monomeren selbst zweckmäßigerweise keine stark nucleophilen Gruppen (wie z. B. $—NH_2$, —SH) enthalten. Weiter können die Bestandteile des Polymerlatex vernetzt sein. Für diese Vernetzung steht Y als Symbol.

Die Härte bzw. die sonstigen relevanten Eigenschaften der Polymerisate aus den individuellen Monomeren ist bekannt, desgleichen der Beitrag zu den Eigenschaften von Copolymerisaten [vgl. US-PS 2 795 564, H. Rauch-Puntigam, T. Völker in « Acryl- und Methacrylverbindungen », Springer-Verlag, Berlin 1967, S. 303-304, T. G. Fox Bull. Am. Phys. Soc. *1*, 123 (1956)].

Im Sinne der schematischen Darstellung seien die in erster Linie für den nicht-hydrophoben bzw. hydrophilen Charakter des Polymerlatex verantwortlichen Bestandteile als B, weitere Bestandteile, deren Auswahl in erster Linie auf die resultierende Härte des Gesamtpolymerisats abgestimmt werden muß, als A bezeichnet, d. h. die Monomeren des Typs A gehören im Sinne der getroffenen Unterscheidung dem nicht-hydrophilen bzw. hydrophoben Typ an.

Der Index p für den monomeren Bestandteil B in dem vorstehend angegebenen Formelschema dient zur Klarstellung des Sachverhalts, daß die primär für die zweckmäßige Härte des Gesamtpolymerisats einzusetzenden Monomeren A anteilmäßig auf die Komponente B abzustimmen ist, so daß p einen Wert von 0 bis zu einem Wert annehmen kann, der einem Anteil von B am Gesamtpolymerisat von 95 Gew.-%, vorzugsweise 0-60 Gew.-%, entspricht. Die für den erfindungsgemäß zu verwendenden Polymerlatex genannten Bedingungen werden z. B. durch Copolymerisate vom Methacrylat- und/oder Acrylattyp erfüllt, wobei der qualitative und der quantitative Anteil so zu bemessen ist, daß die im Anspruch

aufgestellten Kriterien für den Polymerlatex erfüllt sind.

Als nicht hydrophobe bzw. hydrophile Bestandteile B kommen z. B. gegebenenfalls substituierte Methacrylamide und Acrylamide der allgemeinen Formel I

$$CH_2 = \underset{\underset{R_1}{|}}{C} - CONR_3R_4 \quad , \qquad (I)$$

worin $R'_1$ Wasserstoff oder Methyl und $R_3$ und $R_4$ unabhängig voneinander Wasserstoff oder einen Alkylrest mit 1 bis 4 Kohlenstoffatomen bedeuten, also unsubstituierte Amide sowie die mit primären und sekundären Aminen gebildeten Amide sowie Verbindungen, bei denen $R_3$ und $R_4$ zusammen mit dem Stickstoffatom einen, gegebenenfalls ein oder mehrere zusätzliche Heteroatome, insbesondere Stickstoff oder Sauerstoff enthaltenden, gegebenenfalls alkylsubstituierten Ring bilden, infrage. Besonders genannt seien (Meth)-acrylsäureamid, N-Methyl- (bzw. Isopropyl- oder -Butyl)-(meth)-acrylsäureamid, N,N-Dimethyl-(meth)-acrylsäureamid, desweiteren (Meth)acrylsäuremorpholid (Sonderfall, in dem der Stickstoff über $R_3$ und $R_4$ Teil eines Rings ist) und N-Vinylpyrrolidon-2.

Ferner gehören hydroxygruppenhaltige Monomere des Acrylat- oder des Methacrylattyps, insbesondere hydroxygruppenhaltige Ester oder Amide der Acryl- und der Methacrylsäure sowie Alkoxyalkylester- und/oder Amide der Acryl- und der Methacrylsäure zu den hydrophilen Monomeren des Typs B, z. B. Vertreter der allgemeinen Formel II

$$CH_2 = \underset{\underset{R'_1}{|}}{C} - CO - [\, Q - (CH_2)_n \,]_m - OR'_2 \quad , \qquad (II)$$

worin $R'_1$ Wasserstoff oder Methyl, $R'_2$ Wasserstoff oder eine Alkylgruppe mit 1 bis 4 Kohlenstoffatomen, Q Sauerstoff oder eine Gruppe $—NR''_2$, worin $R''_2$ für Wasserstoff oder eine Alkylgruppe mit 1 bis 4 Kohlenstoffatomen steht, n eine ganze Zahl von 1 bis 3, vorzugsweise 2, und m eine ganze Zahl von 1 bis 25 bedeutet, mit der Maßgabe, daß, wenn Q für Sauerstoff steht, n nicht gleich 1 sein soll. Besonders genannt sei das Hydroxyäthylacrylat, Hydroxyäthylmethacrylat, 2-Hydroxyäthyl(meth)-acrylamid, 2-Hydroxypropyl(meth)acrylamid, Monoester der (Meth)-acrylsäure des Glycerins und anderer Polyole.

Weiter fallen unter den Monomerentyp B Sulfoäthylacrylate und Sulfoäthylmethacrylate sowie Sulfoäthylacrylamide und Sulfoäthylmethacrylamide. Ebenfalls als hydrophile Gruppen in die Schale des Latex eingebaut werden können polymerisierbare Säuren, wie (Meth)acrylsäure, Itaconsäure oder Maleinsäure oder polymerisierbare tert. Amine, wie 2-N,N-Dimethylaminoäthyl-(meth)acrylamid bzw. -(meth)acrylsäureester oder 3-N,N-Dimethylaminopropyl-(meth)acrylamid bzw. -ester. Zur Vermeidung einer einseitigen Aufladung der Latexteilchen sollten diese sauren bzw. basischen Gruppen stets gleichzeitig in einem Teilchen vorhanden sein (z. B. Methacrylsäure und 2-N,N-Dimethylaminoäthyl-(methacrylat).

Als Monomere des Typs A kommen nicht oder höchstens begrenzt wasserlösliche Monomere in Frage, wobei der qualitative und der quantitative Anteil so zu bemessen ist, daß das oben aufgeführte Kriterium der Härte des resultierenden Polymerisats erfüllt wird :

a) Ester der Acryl- und/oder der Methacrylsäure, mit $C_1$-$C_{20}$-Alkoholen, insbesondere der Methyl-, Äthyl- sowie die Propyl- und Butylester der Methacrylsäure, sowie der Methyl-, Äthyl- die Propyl- und Butylester und 2-Äthylhexylester der Acrylsäure,

b) copolymerisierbare Monomeren vom Typ der Vinylester, insbesondere Vinylacetat, Vinylpropionat, Vinylbutyrat und Vinylisobutyrat.

Der Anteil der Komponente A am Gesamtpolymerisat kann, weil in Abstimmung mit den übrigen Bestandteilen, innerhalb relativ weiter Grenzen schwanken, beispielsweise von 0 bis 99 Gew.-%, vorzugsweise 20 bis 99 Gew.-%, bezogen auf das Gesamtpolymerisat.

Neben den oben beschriebenen Monomerkomponenten können die erfindungsgemäßen Polymerlatices noch vernetzende Monomere enthalten (Y in der schematischen Darstellung, wobei der Index m null oder eins sein kann ; d. h. der Vernetzer kann fehlen).

Unter « vernetzenden Monomeren » werden wie üblich z. B. solche Monomere verstanden, die zwei oder mehrere reaktive Doppelbindungen im Molekül enthalten, wie z. B. mit der Acrylsäure oder vorzugsweise der Methacrylsäure veresterte Di- oder Polyole sowie Allylverbindungen, wie z. B. Allylmethacrylat, Triallylcyanurat u. a.

Genannt seien z. B. Ethylenglykoldimethacrylat, 1,4-Butandioldimethacrylat, Triglykoldimethacrylat, Trimethylolpropantrimethacrylat.

Der Anteil an Vernetzer — falls vorhanden — richtet sich nach der Hydrophilie des Gesamtpolymerisats. Mit zunehmender Hydrophilie des Latexteilchens kann auch ein zunehmender Anteil an Vernetzer

als vorteilhaft erscheinen.

Er beträgt im allgemeinen zwischen 0 und 50 Gew.-%, vorzugsweise zwischen 0,2 und 15 Gew.-%, bezogen auf das Gesamtpolymerisat.

Der Anteil der funktionellen Monomeren am Gesamtpolymerisat kann — in Abhängigkeit von den konkret verwendeten Monomeren — in weiten Grenzen schwanken. Während z. B. zur kovalenten Fixierung des nucleophile Gruppen tragenden Substrats wenigstens 0,1 % des Monomeren Z'—R—X erforderlich ist, ist der Maximalgehalt dieses Monomeren sehr stark von dem eingesetzten Monomeren selbst abhängig. Für den Fall, daß das reaktive Monomere selbst eine gewisse Hydrophilie aufweist, bzw. unter den Herstellungsbedingungen der Polymerdispersion zu einem gewissen Maße zu einer hydrophilen Verbindung hydrolysiert, so kann der Anteil dieses Monomeren Z—R—X bis zu 99,9 Gew.-% betragen (z. B. im Falle des Glycidyl-methacryllates, Rest = 0,1 % = Vernetzer, z. B. Butandiol-1,4-dimethacrylat).

Als Anhalt für die untere Grenze kann somit 0,1 Gew.-%, bezogen auf das Gesamtpolymerisat, angesehen werden, als Anhalt für die obere Grenze 99,9 Gew.-%, vorzugsweise 1 bis 50 Gew.-%.

Herstellung der Polymer-Latices

Die Herstellung der Latex-Dispersionen kann nach den bekannten Regeln der Emulsionspolymerisation erfolgen, beispielsweise in Anlehnung an DE-OS 18 04 159, DE-OS 19 10 488 und DE-OS 19 10 532, wobei die gewünschte Größe der Latex-Teilchen durch die Emulgatorkonzentration zu Beginn der Polymerisation eingestellt wird. Im allgemeinen liegt die Emulgatorkonzentration zu Beginn der Emulsionspolymerisation zwischen 0,005 und 0,5 Gew.-%, bezogen auf den gesamten Polymerisationsansatz. Die Größe der Latex-Teilchen soll zwischen 0,03 und 6 µm liegen, vorzugsweise zwischen 0,03 und 1 µm. Als Emulgatoren können die bekannten anionischen und nichtionischen Emulgatoren verwendet werden, beispielsweise Fettalkoholsulfate und -sulfonate, -phosphate und phosphonate, Alkalisalze langkettiger Fettsäuren, langkettige Sarkoside sowie oxäthylierte Fettalkohole, substituierte Phenole, die zum Teil sulfiert sein können sowie andere in der Emulsionspolymerisation verwendete Emulgatoren (Houben-Weyl, Methoden der Organischen Chemie, Bd. XIV/I, S. 133-560, G. Thieme-Verlag, *1961*).

Die Verwendung kationischer Tenside empfiehlt sich nur insoweit als sich diese von tertiären oder quartären Ammoniumsalzen ableiten. Desweiteren können auch einpolymerisierbare Emulgatoren verwendet werden.

Als Initiatoren können ebenfalls die allgemein bei Emulsionspolymerisationen üblichen verwendet werden (vgl. J. Brandrup, E. H. Immergut, « Polymer Handbook », second Edition, J. Wiley & Sons ; H. Rauch-Puntigam, Th. Völker, « Acryl- und Methacrylverbindungen », Springer-Verlag 1967). Genannt seien Peroxide, Hydroperoxide, Persäuren und Azoverbindungen, z. B. Kaliumperoxidisulfat, Wasserstoffperoxid u. a. m.

Die Konzentration der Initiatoren liegt in der Regel im üblichen Bereich, beispielsweise bei 0,01 bis 1,0 Gew.-%, bezogen auf die Monomeren.

Der Feststoffgehalt der Dispersion kann je nach Teilchengröße und Hydrophilie der Teilchen zwischen 10 und 60 Gew.-% liegen.

Die Synthese der Latex-Teilchen muß so schonend durchgeführt werden, daß die genannten funktionalen Gruppierungen aus den Monomeren Z'—R—X weitgehend erhalten bleiben, weil nur so eine nachfolgende kovalente Fixierung von Molekülen mit > NH-, —SH- und —COOH-Gruppen möglich ist.

Dabei ist anzumerken, daß der Erhalt der funktionalen nucleophil angreifbaren Gruppen aus den Monomeren Z'—R—X in bzw. an der Oberfläche der Latex-Teilchen um so schwieriger ist, je hydrophiler der Aufbau der Latex-Teilchen ist.

Dies soll am folgenden Beispiel belegt werden :

Bei völlig übereinstimmender Herstellung (Synthesetemperatur : 80 °C, pH : 7,0, 4 h Polymerisationsdauer (Emulsionszulauf). (Zugabe des reaktiven Monomeren Glycidylmethacrylat jeweils nur in der 4. Stunde des insgesamt 4 h dauernden Zulaufs), 1 h Nacherhitzen bei 80 °C findet man den folgenden Oxirangruppengehalt in der Dispersion :

| Bruttozusammensetzung des Polymeren | Oxirangruppengehalt im Latex *) |
|---|---|
| 42,5 % Methylmethacrylat<br>42,5 % Isobutylmethacrylat<br>5 % Ethylenglykoldimethacrylat<br>10 % Glycidylmethacrylat | 71 % |

(Fortsetzung)

| Bruttozusammensetzung des Polymeren | | Oxirangruppengehalt im Latex *) |
|---|---|---|
| 40 | % Methylmethacrylat | |
| 40 | % Isobutylmethacrylat | |
| 5 | % Ethylenglykoldimethacrylat | 15 % |
| 10 | % Glycidylmethacrylat | |
| 5 | % Methacrylamid | |

*) bezogen auf das eingesetzte Glycidylmethacrylat.

Für eine schonende Herstellung der Latex-Teilchen sind die folgenden Punkte maßgeblich :

1. Herstellung der Dispersion in einem pH-Bereich, in dem die Reaktionsgeschwindigkeit des Wassers mit den reaktiven Gruppen am geringsten ist (in der Regel ist dies ein pH von etwa 7).
2. Herstellung bei möglichst niedriger Temperatur.
3. Möglichst kurze Polymerisationsdauer.
4. Abwesenheit von starken Nucleophilen im Latex-Teilchen.

Zu 1. Die Synthese der Latex-Teilchen im neutralen pH-Bereich ist am einfachsten durch eine Pufferung des Systems (z. B. mit Phosphat-Puffer) möglich. Gegebenenfalls kann auf eine Pufferung durch Salzzusatz völlig verzichtet werden, z. B. wenn andere Teile der Rezeptur als Puffersubstanzen wirken können, so z. B. bei Verwendung von Alkalisalzen langkettiger Phosphorsäureester als Emulgatoren oder bei Einsatz des Natriumsalzes der 4,4'-Azobis(cyanovaleriansäure).

Zu 2. und 3. Punkt 2 und 3 beinhalten eine möglichst geringe thermische Belastung der reaktiven Gruppen tragenden Latex-Teilchen. Dabei gilt jedoch folgende Einschränkung : Da vor allem der Gehalt an reaktiven Gruppen auf der Latexoberfläche von Bedeutung ist, ist es durchaus möglich nur die äußere Hülle des Latex-Teilchen unter Zuhilfenahme der oben genannten reaktiven Gruppen herzustellen. So ist z. B. ein Kern-Schale-Aufbau anwendbar, wobei der Latex-Kern völlig frei von reaktiven Monomeren hergestellt wird, dann gelten naturgemäß die Einschränkungen für eine schonende Latex-Herstellung nur für die Schale.

Die Polymerisation wird entweder bei niedrigen Temperaturen (z. B. $< 50\ ^{\circ}C$) mit Hilfe eines Redoxsystems durchgeführt (wobei jedoch darauf zu achten ist, daß Teile des Redox-Systems die reaktiven Gruppen nicht zerstören, z. B. Bisulfit die Oxirangruppen) oder aber mit thermisch zerfallenden Initiatoren oder mit Hilfe eines Redox-Systems bei Temperaturen bis zu 90 °C. Die Polymerisationsdauer sollte 8 Std. nicht überschreiten.

Zu 4. Da der Latex reaktive Gruppen enthält, die eine kovalente Fixierung von > NH-, —SH-, oder —OH-Gruppen haltigen Molekülen ermöglichen soll, ist die Anwesenheit von solchen Gruppen im Latex nur in untergeordnetem Maße möglich. Dies gilt insbesondere für die > NH- und die —SH-Gruppen. Die Anwesenheit von OH-Gruppen im Latex ist weniger kritisch.

Herstellung der oberflächenreichen Gebilde

Die erfindungsgemäßen Polymerlatices werden bevorzugt auf geeignete Träger aufgebracht. In erster Linie eignen sich inerte (im allgemeinen nicht-wasserlösliche) Träger, insbesondere feste Träger, vorzugsweise mit möglichst großer Oberfläche. Unter praktischen Gesichtspunkten bieten sich insbesondere poröse Körper als Träger an, z. B. auch geschäumte Materialien, Schwämme, weiter Faserstrukturen, Vliese usw. Geeignet sind sowohl anorganische als organische Trägermaterialien.

Genannt seien beispielsweise Träger auf Siliciumdioxid- bzw. Silikatbasis, insbesondere auch feinverteiltes Siliciumdioxid, z. B. in Form von Gelen bzw. als Aerosil®, ferner Träger auf der Basis von Aluminiumoxid und/oder anderen Metalloxiden und auf Basis von Tonen, wie z. B. Füllerden, Bleicherden usw. und Keramik. Ferner fein verteilte anorganische Pigmente, wie Titandioxid, Baryt, ferner Kreide, Talkum, Gips, Bimsstein, Glas, Aktivkohle, rostfreier Stahl usw. Außerdem geeignet erscheint z. B. ein wabenförmig aufgebautes Material auf Cordierit-Basis ($Mg_2Al_4Si_5O_{18}$).

Als Träger organischer Herkunft bieten sich sowohl (modifizierte) Naturprodukte als auch synthetische Materialien polymerer Natur an. Aus dem Bereich der Naturprodukte sind insbesondere Fasereiweißstrukturen, wie z. B. Wolle und solche auf Kohlehydratbasis (Cellulose, z. B. Zellstoff, Stärke, insbesondere vernetzte Dextrane, usw.) zu nennen. Ebenso eignen sich Materialien auf der Basis synthetischer Polymerer, wie z. B. Polyamide, Polyester, Polyurethan, Polyacrylnitril, Polyimidschäume. Die genannten Stoffe bieten besonders interessante Aspekte, wenn sie als Flächengebilde, wie z. B. Vliese, Watten oder (nicht geleimtes) Papier oder entsprechende dreidimensionale makroporöse Körper

zur Anwendung kommen.

Die oben beschriebenen, reaktiven Latex-Teilchen können in an sich bekannter Weise durch Tränken, Sprühen oder andere Techniken auf oberflächenreiche Gebilde, wie z. B. Papier, Watte, Vlies u. v. a. auch anorganische Trägermaterialien aufgebracht werden.

Dabei kommen zwei verschiedene Bindungsmechanismen zur Anwendung :

α. Der Latex ist bei der Auftragungstemperatur filmbildend

In diesem Falle wählt man eine Latexmasse, die kleiner ist als die Masse des Trägermaterials (fest/fest), um die Gesamtoberfläche des Trägermaterials nicht zu verkleinern.

β. Der Latex ist bei der Auftragungs- und/oder der Anwendungstemperatur nicht filmbildend

In diesem Fall kann je nach Oberflächenbeschaffenheit des Trägermaterials das Verhältnis Latex (Festsubstanz) : Trägersubstanz von 1 : 100 bis 100 : 1 reichen.

Gegebenenfalls kann in diesem Falle auf einen festen Träger völlig verzichtet werden. Dazu ist es erforderlich den Latex durch Sprühtrocknung, Gefriertrocknung oder durch Ausfällen (z. B. durch Natriumsulfat) bzw. durch weitere Methoden, wie Koagulation infolge von Hitzeeinwirkung, Ausfrieren, Einwirkung von Lösungsmitteln, zu agglomerieren, so daß eine möglichst große innere Oberfläche erhalten bleibt.

Sofern die Verbindung der einzelnen Latex-Teilchen untereinander nicht durch Filmbildung erfolgt, kann ihre Verknüpfung untereinander bzw. zum Träger durch kovalente Bindungen geschehen. Im Falle der Oxirangruppen enthaltenden Latex-Teilchen kann diese Bindung z. B. durch Reaktion des Oxiranrings mit den OH-Gruppen benachbarter Latex-Teilchen bzw. mit OH-Gruppen des Trägermaterials erfolgen. Gegebenenfalls kann diese kovalente Verknüpfung der Latex-Teilchen durch den Einsatz von multifunktionellen Nucleophilen, z. B. Polyaminen, verstärkt werden. Die Fixierung der Latexteilchen auf den Träger oder zueinander kann jedoch auch über Nebenvalenzbindungen oder durch Verkitten mit untergeordneten Mengen einer weichen, filmbildenden Substanz, z. B. mit Latexteilchen niedrigerer Glastemperatur erfolgen. Diese weichen Latexteilchen können ebenfalls funktionelle Gruppen enthalten.

Eine besonders bevorzugte Ausführungsform besteht darin, zum Ausfällen die funktional und/oder morphologisch definierten biologisch wirksamen Einheiten selbst einzusetzen, z. B. kann man ein zu bindendes Protein, z. B. ein Enzym, selbst als multifunktionellen Vernetzer einsetzen. Dieses Verfahren ist vor allem bei besonders kleinen Latex-Teilchen anwendbar.

Der Einsatz der oberflächenreichen Gebilde als Katalysatoren (z. B. nach Fixierung von Enzymen) erfolgt je nach Art des vorgegebenen Trägermaterials (z. B. im Festbett). Der Einsatz der sprühgetrockneten bzw. mit Salz oder in Gegenwart von Enzymen ausgefällten Dispersionen erfolgt vorzugsweise im Batch oder im Fließbett. Der hohen Porosität und sehr hohen katalytischen Aktivität dieser Materialien steht eine nur geringe mechanische Festigkeit gegenüber. Für den Fall, daß das zu bindende, nucleophile Gruppen enthaltende Substrat nicht bereits bei der Agglomeration der Latex-Teilchen anwesend war, erfolgt die Umsetzung dieser Substrate mit den oberflächenreichen, reaktiven Gebilden unter den üblichen Bedingungen (z. B. Bindung des Enzyms Trypsin an einen oxiran-gruppenhaltigen Träger in einem unimolaren Phosphatpuffer (pH 7,5) in 72 h bei 23 °C). Als Substrate können allgemein funktional und/oder morphologisch definierte, biologisch wirksame Einheiten oder Substanzen dienen. Genannt seien z. B. Proteine allgemein, insbesondere Enzyme, Blutbestandteile und Blutinhaltsstoffe (Blutfaktoren), z. B. Albumine, Immunoglobuline, Faktoren der Blutgerinnung, Zellmembranproteine, Peptidhormone u. ä. Weiterhin seien erwähnt hochmolekulare, biogene Substrate, die gegebenenfalls durch Farbstoffe markiert sein können, z. B. zur Anwendung in der Diagnostik. Neben dem Einsatz als Katalysator ist vor allem auch ein Einsatz in der (Affinitäts)-Chromatographie und allgemein im Bereich der Diagnostik möglich.

Bei dem Einsatz — z. B. als Diagnosepapier — kann es von Vorteil sein, einen — (z. B. pH- oder Redox-sensitiven) Farbstoff physikalisch oder kovalent mit einzulagern. Damit kann z. B. bei gleichzeitiger Anwesenheit eines Substrats der entsprechende Reaktionspartner, z. B. ein Enzym, durch eine Farbänderung nachgewiesen werden. Zum Zwecke der Diagnose kann der reaktive Latex auf Papierstreifen oder Teststäbchen aus beliebigem Trägermaterial aufgebracht werden. Die mit dem Reagenz (z. B. einem Enzymsubstrat und gegebenenfalls zusätzlich mit einem Farbstoff) umgesetzten Teststreifen oder Teststäbchen sind dann im trockenen Zustand unter Einhaltung bestimmter Temperaturbedingungen beliebig lagerfähig.

Der eigentliche Test kann zu gegebener Zeit durch einfaches Eintauchen des Teststreifens oder Stäbchens in ein den Reaktionspartner (z. B. ein Enzym) enthaltendes (Medium (z. B. Urin, Blutserum etc.) erfolgen. Neben diesen spezifischen Diagnoseanwendungen kann das oberflächenreiche System ganz generell als bioaffiner Indikator Verwendung finden. Bei einer Verwendung als Katalysator, z. B. als Biokatalysator, können die reaktiven Latices in zwei prinzipiell verschiedenen Versionen zum Einsatz kommen. Bei Anwendung im Festbett wird der Latex im allgemeinen an eines der oben beschriebenen Trägermaterialien fixiert. Die Umsetzung mit dem Katalysator kann vor oder nach dieser Fixierung

stattfinden. Am interessantesten sind wegen ihrer hohen Spezifität und Selektivität Enzyme (es können aber auch einfacher aufgebaute und unspezifisch wirkende Gruppen (z. B. quartäre Ammoniumverbindungen oder Imidazol u. a. Heterocyclen zur Katalyse einer Hydrolyse) zur Anwendung kommen).

Die erfindungsgemäßen reaktiven oberflächenreichen Gebilde sind zur Immobiliserung aller Klassen von Enzymen geeignet, beispielsweise zur Fixierung von Oxidoreduktasen, Transferasen, Hydrolasen, Lyasen, Isomerasen und Ligasen.

Im Falle einer Verwendung im Fließbett sind neben zerkleinerten, auch für das Festbett geeigneten Träger-Katalysator-Kombinationen in erster Linie trägerfreie Latexaggregate einsetzbar, die als Pulver, Aufschlämmung oder in einer anderen grobdispersen Form zur Anwendung kommen können. Die Umsetzung mit dem Enzymsubstrat kann am Originallatex selbst oder nach dessen Agglomeration erfolgen. Im übrigen sind dieselben Katalysatoren einsetzbar wie sie weiter oben im Falle der Festbettkatalyse beschrieben worden sind.

Falls für eine chemische Reaktion mehrere Enzyme in das Reaktionsgeschehen eingreifen müssen, besteht häufig das Problem, daß diese Enzyme nicht nebeneinander existenzfähig sind. Im Falle der kovalenten Bindung an die beanspruchten Trägermaterialien lassen sich solche Unverträglichkeiten häufig herabsetzen oder ganz ausschließen. So ist es bei dem erfindungsgemäßen Verfahren durchaus möglich zwei oder mehrere, verschiedene Enzyme zu fixieren. So eignen sich die erfindungsgemäßen, oberflächenreichen Systeme zur Fixierung von therapeutisch verwendbaren Enzymen, die z. B. oral angewendet werden können (z. B. Proteasen und/oder Lipasen und/oder Amylasen). Es kann aber auch vorteilhaft sein, Enzymkombinationen direkt im Zellverband zur Anwendung zu bringen, d. h. ganze Mikroorganismen zu immobilisieren. Dies kann besonders vorteilhaft mit den beanspruchten, reaktiven Latices geschehen. Durch Menge und Teilchengröße der Latices ist die Substratzugänglichkeit eines solchen Systems besonders gut zu steuern.

Auf diese Weise können demzufolge auch noch höhermolekulare Substrate umgesetzt werden.

Für eine solche Einbettung oder Fixierung kommen besonders in Frage :

1. Viren, prokariotische und eukariotische Zellen und deren Untereinheiten, siehe 2., sowie Zellhybride, wie sie z. B. zur Herstellung von monoclonalen Antikörpern benutzt werden.

2. Außerdem eignen sich die erfindungsgemäßen reaktiven oberflächenreichen Gebilde zur Immobilisierung von Zellorganellen, insbesondere Mitochondrien, Mikrosomen, Membranteilen, Zellkernen und deren Untereinheiten.

Die Anwendung der erfindungsgemäßen Latex-Trägerkombinationen in der (Affinitäts) chromatographie erfolgt völlig analog wie bei der Katalysatoranwendung beschrieben. Lediglich, die an den reaktiven Latex zu bindenden reaktiven Moleküle sind auf den spezifischen Verwendungszweck ausgerichtet. Eine Anwendungsmöglichkeit der oberflächenreichen Systeme gemäß der vorliegenden Erfindung ist die Anwendung als bioaffines Sorbens. Dabei können durchaus Überschneidungen auftreten, etwa in der Art, daß ein gebundenes Enzym, das als Katalysator eingesetzt werden kann, Anwendung findet in der chromatographischen Reinigung eines Enzyminhibitors.

Eine besonders interessante Einsatzmöglichkeit bieten die Träger bei der Beseitigung von Spuren toxischer Stoffe (z. B. Blutwäsche, Wasseraufbereitung etc.). Es kann aber auch das reaktive oberflächenreiche System eingesetzt werden. In diesem Falle ist die Entfernung von nucleophilen Verunreinigungen aus wäßrigem Medium möglich, z. B. die Entfernung von toxischen Aminen, Mercaptanen und anderen Schadstoffen aus wäßrigen Medien. Darüber hinaus kann das erfindungsgemäße, oberflächenreiche System mit multifunktionellen Substanzen umgesetzt werden, die mindestens eine nucleophile Gruppe (zur Fixierung am Latex) und mindestens eine weitere funktionelle Gruppe aufweisen, die zur spezifischen Wechselwirkung mit in wäßrigem Medium befindlichen Substanzen oder funktionellen Einheiten geeignet ist. Gruppen mit der genannten spezifischen Wechselwirkung können beispielsweise Komplexbildner sein ; genannt sei z. B. das Umsetzungsprodukt der Aminodiessigsäure mit einem oxiranhaltigen Latex u. ä.

Weitere Einsatzmöglichkeiten der erfindungsgemäßen reaktiven oberflächenreichen Systeme liegen in der Anwendung als stationäre Phase in der präparativen organischen Chemie. So kann z. B. ein auf diese Weise erhaltener S-S-Brückenhaltiger Latex als schonendes Oxidationsmittel verwendet werden, wobei die Entfernung des entstehenden Mercaptans entfällt. Von ganz besonderem Interesse ist die Anwendung als stationäre Phase in der präparativen Peptidsynthese. Dabei kann einerseits das zu synthetisierende Peptid an dem oberflächenreichen System aufgebaut werden, es kann aber auch das oberflächenreiche System aktivierte Reaktionspartner, z. B. Kupplungsaktive Aminosäuren oder Kupplungsagentien, z. B. nur schwer in Lösung überzuführende Carbodiimide aufnehmen. Im letzten Falle bleibt das aufzubauende Peptid in der wäßrigen Phase gelöst. Prinzipiell sind für chromatographische Zwecke sowohl die an feste Träger gebundene Latex-Wirkstoff-Kombination als auch die lose aggregierte Latex-Wirkstoff-Kombination (Säule) einsetzbar.

Die nachstehenden Beispiele stellen eine Auswahl zur Erläuterung des erfindungsgemäßen Immobilisierungsprinzips dar.

Beispiel 1

Herstellung einer oxirangruppenhaltigen Dispersion

In einem Polymerisationsgefäß, ausgestatt mit Rückflußkühler, Rührer und Thermostat wird eine Lösung aus

10 g Phosphatpufferlösung pH 7,0 (Titrisol, Merck)
0,4 g Natriumsalz der 4,4'-Azobis-(4-cyanovaleriansäure)
0,3 g Natriumlaurylsulfat
555 g destilliertem Wasser vorgelegt

und auf 80 °C erwärmt.
In diese Vorlage tropft man ebenfalls bei 80 °C innerhalb von 4 Stunden eine Emulsion, hergestellt aus :

360 g Methylmethacrylat
210 g Butylacrylat
30 g Glycidylmethacrylat
2 g Natriumsalz der 4,4'-Azobis-(4-cyanovaleriansäure)
3 g Natriumlaurylsulfat und
840 g destilliertem Wasser.

Anschließend wird noch weitere 2 Stunden bei 80 °C gerührt, danach wird auf Raumtemperatur abgekühlt und filtriert. Man erhält eine koagulatfreie Dispersion. Feststoffgehalt ca. 30 %, pH-Wert : 7,3, Viskosität 2 mPa · s.

Beispiel 2

Herstellung einer oxirangruppenhaltigen Dispersion

In einem Polymerisationsgefäß, ausgestattet mit Rückflußkühler, Rührer und Thermostat wird eine Lösung aus

50 g Phosphatpufferlösung pH 7,0 (Titrisol, Merck)
0,3 g Natriumlaurylsulfat
0,3 g Natriumsalz der 4,4'-Azobis-(4-cyanovaleriansäure)
515 g destilliertem Wasser vorgelegt und auf

80 °C erwärmt.
In diese Vorlage tropft man ebenfalls bei 80 °C innerhalb von 4 Stunden eine Emulsion, hergestellt aus :

300 g Methylmethacrylat
210 g Butylacrylat
90 g Glycidylmethacrylat
2 g Natriumsalz der 4,4'-Azobis-(4-cyanovaleriansäure)
3 g Natriumlaurylsulfat und
840 g destilliertem Wasser.

Anschließend wird noch 90 min bei 80 °C gerührt, danach wird auf Raumtemperatur abgekühlt und filtriert. Man erhält eine gut filtrierbare, koagulatfreie Dispersion. Feststoffgehalt ca. 30 %, pH-Wert : 7,1, Viskosität 1 mPa · s.

Beispiel 3

Herstellung einer oxirangruppenhaltigen Dispersion

In einem Polymerisationsgefäß, ausgestattet mit Rückflußkühler, Rührer und Thermostat wird eine Lösung aus

6,5 g Natriumlaurylsulfat
0,6 g Natriumsalz der 4,4'-Azobis-(4-cyanovaleriansäure)
10,0 g Phosphatpufferlösung pH 7,0 (Titrisol, Merck)
600,0 g destilliertem Wasser

vorgelegt und auf 80 °C erwärmt.

In diese Vorlage tropft man bei 80 °C innerhalb von 3 h eine Emulsion, hergestellt aus :

18 g Methacrylamid
11 g Äthylenglykoldimethacrylat
150 g Methylmethacrylat
180 g Glycidylmethacrylat
1,5 g Natriumlaurylsulfat
2,0 g Natriumsalz der 4,4'-Azobis-(4-cyanovaleriansäure) und
900 g destilliertem Wasser.

Anschließend wird noch weitere 30 min bei 80 °C gerührt, danach wird auf Raumtemperatur abgekühlt und filtriert. Man erhält eine gut filtrierbare, koagulatfreie Dispersion, Feststoffgehalt : 19,4 %, pH-Wert : 7,7, Viskosität 2 mPa · s.

## Beispiel 4

Herstellung einer oxirangruppenhaltigen Dispersion

Man verfährt wie in Beispiel 3, dosiert jedoch eine Emulsion mit veränderter Monomerzusammensetzung zu.

Monomerzusammensetzung :

18 g Methacrylamid
36 g Äthylenglykoldimethacrylat
125 g Methylmethacrylat
180 g Glycidylmethacrylat

Hilfsstoffe, Polymerisationsdauer und Polymerisationstemperatur wie in Beispiel 3 beschrieben.

Es resultiert eine gut filtrierbare, koagulatfreie Dispersion.

Feststoffgehalt : 19,7 %, pH-Wert : 7,6, Viskosität : 1 mPa · s.

## Beispiel 5

Immobilisierung des Enzyms Ribonuclease durch Umsetzung mit Latex gemäß Beispiel 4

100 mg Ribonuclease E. C. 2.7.7.16 (aus Pankreas, Merck, Artikel Nr. 24570) werden in 1 ml 0,05 M Phosphatpuffer pH 7,5 gelöst. Zu dieser Lösung gibt man unter Rühren 1 ml der Dispersion gemäß Beispiel 4. Anschließend wird 3 Tage bei 23 °C stehengelassen.

Zur Aufarbeitung wird dreimal in jeweils 50 ml 1 M NaCl-Lösung aufgeschlämmt und anschließend zentrifugiert. Danach wird dieser Waschvorgang noch zweimal mit 50 ml 0,05 M Phosphatpuffer wiederholt.

Ausbeute : 1,1 g Feuchtsubstanz.

Die Ermittlung der Enzymaktivität erfolgte durch alkalimetrische Titration bei 37 °C und pH 7,5, mit RNA als Substrat.

| Verwendung | Feuchteinwaage [g] | Aktivität *) U/g Feuchteinwaage |
|---|---|---|
| 1 | 1,10 | 101 |
| 2 | 1,75 | 54,8 |
| 3 | 1,74 | 51,0 |
| 4 | 1,68 | 55,0 |
| 5 | 1,71 | 51,9 |

*) 1 U entspricht 1 µmol/min, gemessen anhand der Anfangsgeschwindigkeit.

Beispiel 6

Immobilisierung des Enzyms Trypsin durch Umsetzung mit dem Latex gemäß Beispiel 4

Man verfährt wie im Beispiel 5, verwendet jedoch als Enzym 100 mg Trypsin E. C. 3.4.4.4 (vom Rind, Merck, Artikel Nr. 24579).

Die Ermittlung der Enzymaktivität erfolgte durch alkalimetrische Titration bei 37 °C und pH 7,5 (BAEE) und pH 8,0 (Casein).

| Verwendung | Aktivität [U/g] *) (Substrat: BAEE) | Aktivität [U/g] *) (Substrat: Casein) |
|---|---|---|
| 1 | 174 | 42,5 |
| 2 | 163 | 26,7 |
| 3 | 161 | 23,3 |
| 4 | 161 | 23,3 |

*) Aktivitäten jeweils bezogen auf die Feuchteinwaage, 1 U entspricht 1 µmol/min, gemessen anhand der Anfangsgeschwindigkeit

BAEE = Nα-Benzoyl-L-argininethylesterhydrochlorid

Beispiele 7-11

Immobilisierung des Enzyms Trypsin durch Umsetzung mit dem Latex gemäß Beispiel 4

Man verfährt wie im Beispiel 6, variiert jedoch das Verhältnis Enzym/Latex.

Aktivität

| Beispiel Nr. | Einwaage 2) Trypsin [mg] | Einwaage Latex-Feststoff [mg] | Verhältnis Trypsin/ Latex-Feststoff | Aktivität *) [U/g] (Substrat:Casein) |
|---|---|---|---|---|
| 7 | 20 | 200 | 1 : 10 | 3,7 |
| 8 | 40 | 200 | 1 : 5 | 13,8 |
| 9 | 80 | 200 | 1 : 2,5 | 22,2 |
| 6 | 100 | 200 | 1 : 2 | 23,3 |
| 10 | 160 | 200 | 1 : 1,25 | 26,4 |
| 11 | 200 | 200 | 1 : 1 | 22,3 |

*) Aktivität jeweils bei der 3. Verwendung

Beispiel 12

Immobilisierung des Enzyms PC-Amidase durch Umsetzung mit dem Latex gemäß Beispiel 4

Man verfährt wie im Beispiel 5, verwendet jedoch als Enzym 100 mg Penicillin-Amidase (Escherichia coli). E. C. 3.5.1.11.

Die Ermittlung der Enzymaktivität erfolgt durch Messung bei 37 °C und pH 7,8. (Substrat : Penicillin-Kalium G).

Aktivitätsmessung

| | |
|---|---|
| 1. Verwendung | 42,7 U/g Feuchteinwaage |
| 2. Verwendung | 42,0 U/g Feuchteinwaage |
| 3. Verwendung | 41,6 U/g Feuchteinwaage |

Beispiel 13

Immobilisierung des Enzyms Ribonuclease durch Umsetzung mit dem Latex gemäß Beispiel 3

Man verfährt wie im Beispiel 5, verwendet jedoch zur Vernetzung des Enzyms den Latex gemäß Beispiel 3.

Aktivitätsmessung

| | |
|---|---|
| 1. Verwendung | 68,5 U/g Feuchteinwaage |
| 2. Verwendung | 61,8 U/g Feuchteinwaage |
| 3. Verwendung | 61,8 U/g Feuchteinwaage |
| 4. Verwendung | 61,0 U/g Feuchteinwaage |

Beispiel 14

Fixierung des Enzyms RNase an ein durch Tränken mit einer oxirangruppenhaltigen Dispersion aktiviertes Papier

100 ml der Dispersion gemäß Beispiel 1 werden mit 500 ml destilliertem Wasser verdünnt. Mit dieser etwa 5 %igen Dispersion tränkt man ein ca. 100 $cm^2$ großes Stück Papier (Whatman Medium flow). Anschließend wird abgepreßt, eine Stunde bei Raumtemperatur belassen und danach 30 min bei 80 °C getrocknet. Das so behandelte Papier enthält 20 g Polymerisatfeststoff pro $m^2$. Bei Temperaturen unter — 15 °C kann dieses reaktive Papier wenigstens 12 Monate gelagert werden.

Fixierung des Enzyms Ribonuclease

100 mg Ribonuclease (aus Pankreas, Merck, Artikel Nr. 24570) werden in 2 ml 0,05 M Phosphatpuffer (pH 7,5) gelöst. Mit dieser Lösung tränkt man das mit dem oxirangruppenhaltigen Latex behandelte Papier und läßt anschließend 72 h bei 23 °C stehen. Danach wird abgepreßt und dreimal mit 1n NaCl- und zweimal mit 0,05 M Phosphatpufferlösung (pH 7,5) gewaschen.

Aktivitätsmessung (3. Verwendung) : 2 U/g Feuchtpapier Bedingungen : 37 °C, pH 7,5

Beispiel 15

Man verfährt wie im Beispiel 14, verwendet jedoch das Enzym Trypsin. E. C. 3.4.4.4.

Aktivitätsmessung (3. Verwendung) : Substrat BAEE, pH 7,5, 37 °C, 2,5 U/g Feuchtpapier.

Beispiel 16

Man verfährt wie im Beispiel 14, verwendet jedoch zum Tränken des Papiers eine konzentriertere Dispersion (100 ml der Dispersion gemäß Beispiel 1, verdünnt mit 200 ml destilliertem Wasser).

Trocknungsbedingungen : 12 h bei 25 °C im Umlufttrockenschrank.

Polymerisatfeststoff/$m^2$ Papier = 33 g

Anschließend Fixierung des Enzyms Trypsin wie im Beispiel 14 für das Enzym RNase beschrieben.

Aktivitätsmessung (3. Verwendung) : 4,1 U/g Feuchtpapier Substrat BAEE, pH 7,5, 37 °C.

Beispiel 17

Man verfährt wie im Beispiel 16, verwendet zur Tränkung den Latex gemäß Beispiel 2 (Verdünnung wie im Beispiel 16 : 100 ml Dispersion in 200 ml destilliertem Wasser).

Polymerisatfeststoff/$m^2$ Papier = 27 g

Aktivitätsmessung (3. Verwendung) : 7 U/g Feuchtpapier Substrat BAEE, pH 7,5, 37 °C

Beispiel 18

100 ml der Dispersion gemäß Beispiel 1 wird mit 200 ml destilliertem Wasser verdünnt, damit wird eine Watte (Dicke 2 mm) besprüht, anschließend wird 12 h bei Raumtemperatur getrocknet.

Polymerisatfeststoff/$m^2$ Watte = 39 g

Aktivitätsmessung (3. Verwendung, Enzym RNase) = (aus Pankreas, Merck, Artikel Nr. 24570) 2,1 U/g Feuchtwatte

Messbedingungen wie im Beispiel 14 beschrieben.

Beispiel 19

Man verfährt wie im Beispiel 14 : Tränkung des Papiers mit der oxirangruppenhaltigen Dispersion gemäß Beispiel 1, Trocknung, Umsetzung mit dem Enzym Ribonuclease (aus Pankreas, Merck, Artikel Nr. 24570). Das 72 h mit Enzymlösung behandelte Papier wird jedoch ohne weitere Reinigung 24 h zusätzlich mit einer 0,005 %igen Lösung von 4'-Amino-azobenzol-2-carbonsäure in 0,05 M Phosphatpufferlösung behandelt, anschließend wird dreimal mit 1n NaCl- und zweimal mit 0,05 M Phosphatpufferlösung (pH 7,5) gewaschen. Zur Verwendung als Indikator (Substratnachweis) wird das Papier noch zweimal mit 1n NaCl-Lösung gewaschen. Bei Anwesenheit eines Substrats zeigt das Papier Farbumschlag von gelb nach orange.

Aktivität des Teststreifens bei pH 7,5 (37 °C) : 1,5 U/g Feuchtgewicht

Beispiel 20

Immobilisierung von Escherichia coli durch Umsetzung mit dem Latex gemäß Beispiel 4.

Zu 10 ml einer 20 %igen Zellsuspension von Escherichia coli in physiologischer Kochsalzlösung werden 10 ml der Dispersion gemäß Beispiel 4 gegeben. Anschließend läßt man 24 Stunden bei Raumtemperatur stehen. Es resultiert ein Netzwerk, das sich gut durch Zentrifugation reinigen läßt.

Aktivitäten

Substrat : Penicillin-Kalium (2 %ig), 37 °C

| Verwendung | Aktivität [U/g Feuchtkatalysator] |
|---|---|
| 1 | 118 |
| 2 | 66 |
| 3 | 55 |
| 4 | 53 |

Damit sind die Aktivitäten des immobilisierten Escherichia coli durchaus vergleichbar mit denen des nicht immobilisierten Escherichia coli :

| Verwendung | Aktivität |
|---|---|
| 1 | 128,5 |
| 2 | 132,5 |
| 3 | 132,5 |

**Patentansprüche**

1. Oberflächenreiche Systeme mit reaktiven Einheiten zur Bindung von nucleophile Gruppen enthaltenden Substraten, dadurch gekennzeichnet, daß die reaktiven Einheiten zur Bindung der die nucleophilen Gruppen enthaltenden Substrate von der Herstellung her Bestandteil eines durch Emulsionspolymerisation hergestellten Polymerlatex sind, der aus 0,03 bis 6 µm großen Latexteilchen aufgebaut ist und der Polymerlatex selbst zu einem oberflächenreichen System aggregiert und/oder an einem oberflächenreichen Trägermaterial fixiert ist.

2. Oberflächenreiche Systeme gemäß Anspruch 1, dadurch gekennzeichnet, daß die Aggregation der Polymerlatices zu einem oberflächenreichen System durch Sprühtrocknung unterhalb der minimalen Filmbildungstemperatur (MFT) des individuellen Polymerlatex vorgenommen wird.

3. Oberflächenreiche Systeme gemäß Anspruch 1, dadurch gekennzeichnet, daß die Aggregation der Polymerlatices zu einem oberflächenreichen System durch Gefriertrocknung erfolgt.

4. Oberflächenreiche Systeme gemäß Anspruch 1, dadurch gekennzeichnet, daß die Aggregation der Polymerlatices zu einem oberflächenreichen System durch thermische Koagulation, Ausgefrieren oder durch Fällen mit Elektrolyten oder Lösungsmitteln erfolgt.

5. Oberflächenreiche Systeme gemäß Anspruch 1, dadurch gekennzeichnet, daß die Aggregation der Polymerlatices zu einem oberflächenreichen System durch Ausfällen mit dem die nucleophilen Gruppen enthaltenden Substrat erfolgt.

6. Oberflächenreiche Systeme gemäß den Ansprüchen 1 und 5, dadurch gekennzeichnet, daß das die nucleophilen Gruppen enthaltende Substrat Protein-Charakter hat.

7. Oberflächenreiche Systeme gemäß den Ansprüchen 1 und 5, dadurch gekennzeichnet, daß die zum Ausfällen benutzten, nucleophilen Gruppen enthaltenden Substrate aus funktional und/oder morphologisch definierten, biologisch wirksamen Einheiten bestehen.

8. Oberflächenreiche Systeme gemäß den Ansprüchen 1, 5, 6 und 7, dadurch gekennzeichnet, daß das die nucleophilen Gruppen enthaltende Substrat ein Enzym ist.

9. Oberflächenreiche Systeme gemäß den Ansprüchen 1 und 8, dadurch gekennzeichnet, daß das die nucleophilen Gruppen enthaltende Substrat aus mehreren, unterscheidbaren Enzymen besteht.

10. Oberflächenreiche Systeme gemäß den Ansprüchen 1 und 7, dadurch gekennzeichnet, daß die zum Ausfällen benutzte, nucleophile Gruppen enthaltenden Substrate aus Mikroorganismen oder deren Untereinheiten, Viren, eukariotischen oder prokariotischen Zellen und/oder deren Untereinheiten oder Zellhybriden oder Zellorganellen, wie Mitochondrien, Mikrosomen, Membranteile, Zellkerne oder deren Untereinheiten, bestehen.

11. Oberflächenreiche Systeme gemäß Anspruch 1 und 2-4, dadurch gekennzeichnet, daß bei der Aggregation der Polymerlatices zu einem oberflächenreichen Gebilde ein oder mehrere Arten der die nucleophilen Gruppen enthaltenden Substrate bereits anwesend sind.

12. Oberflächenreiche Systeme gemäß Anspruch 1, dadurch hergestellt, daß auf ein oberflächenreiches Trägermaterial eine Latex-Dispersion aufgebracht wird, deren Latexteilchen als Bestandteile reaktive Gruppen zur Bindung der die nucleophilen Gruppen enthaltenden Substrate aufweisen und der Latex am Trägermaterial fixiert wird.

13. Oberflächenreiche Systeme gemäß den Ansprüchen 1 und 12, dadurch gekennzeichnet, daß der auf das oberflächenreiche Trägermaterial aufzubringende Latex filmbildend ist und durch einfaches Auftrocknen auf dem oberflächenreichen Trägermaterial fixiert wird.

14. Oberflächenreiche Systeme gemäß den Ansprüchen 1 und 12, dadurch gekennzeichnet, daß der zu fixierende Latex an sich nicht filmbildend ist und durch Beifügung geringfügiger Mengen eines filmbildenden Latex oder mittels der reaktiven Einheiten bzw. weiterer funktioneller Gruppen oder reaktiver Verbindungen am Trägermaterial fixiert wird.

15. Oberflächenreiche Systeme gemäß den Ansprüchen 1 und 12, dadurch gekennzeichnet, daß das oberflächenreiche Trägermaterial im wesentlichen aus organischem Material aufgebaut ist.

16. Oberflächenreiche Systeme gemäß Anspruch 15, dadurch gekennzeichnet, daß das organische Material aus Vinylpolymeren, Kohlehydraten, Proteinen, Polyaminosäuren, Polyamiden oder Polyestern aufgebaut ist.

17. Oberflächenreiche Systeme gemäß den Ansprüchen 12, 15 und 16, dadurch gekennzeichnet, daß das oberflächenreiche Trägermaterial ein Faservlies darstellt.

18. Oberflächenreiche Systeme gemäß den Ansprüchen 12, 15 und 16, dadurch gekennzeichnet, daß das oberflächenreiche Trägermaterial eine Watte darstellt.

19. Oberflächenreiche Systeme gemäß den Ansprüchen 12, 15 und 16, dadurch gekennzeichnet, daß das oberflächenreiche Trägermaterial geschäumt ist.

20. Oberflächenreiche Systeme gemäß den Ansprüchen 1 und 12, dadurch gekennzeichnet, daß das oberflächenreiche Trägermaterial aus anorganischem Material aufgebaut ist.

21. Oberflächenreiche Systeme gemäß Anspruch 20, dadurch gekennzeichnet, daß das anorganische Trägermaterial in Vliesform oder in Form eines porösen Körpers vorliegt.

22. Oberflächenreiche Systeme gemäß Anspruch 20, dadurch gekennzeichnet, daß das anorganische Material aus Siliciumdioxid bzw. Silikaten, Glas, Aluminiumoxid und/oder andere Metalloxiden, Tonen, Sand, Keramik, Kohle, nicht-rostendem Stahl, besteht.

23. Oberflächenreiche Systeme gemäß den Ansprüchen 1 bis 22, dadurch gekennzeichnet, daß der Polymerlatex aus radikalisch polymerisierbaren Monomeren aufgebaut ist.

24. Oberflächenreiche Systeme gemäß den Ansprüchen 1 und 23, dadurch gekennzeichnet, daß der Polymerlatex auf Basis von Acrylsäure- und/oder Methacrylsäurederivaten und/oder Styrol und/oder Vinylacetat aufgebaut ist.

25. Oberflächenreiche Systeme gemäß den Ansprüchen 1 bis 24, dadurch gekennzeichnet, daß die reaktiven Einheiten als Bestandteile eines Polymerlatex solche sind, die mit den nucleophile Gruppen enthaltenden Substraten in wäßriger Lösung und im pH-Bereich von 5-10 unter Ausbildung einer kovalenten Bindung reagieren.

26. Oberflächenreiche Systeme gemäß Anspruch 25, dadurch gekennzeichnet, daß die reaktiven Einheiten Oxiran-, Keto-, Formyl-, Sulfonsäurehalogenid-, Thioisocyanatgruppen, aktivierte Carbonsäureester und/oder Carbonsäureanhydride oder aktivierte Doppelbindungen darstellen.

27. Oberflächenreiche Systeme gemäß den Ansprüchen 1 bis 26, dadurch gekennzeichnet, daß sie zusätzlich noch einen, gegebenenfalls pH- oder redoxsensitiven Farbstoff enthalten.

28. Verwendung der oberflächenreichen Systeme gemäß den Ansprüchen 1 bis 4 und 12, dadurch gekennzeichnet, daß man die am Trägermaterial fixierten Latices mit einem nucleophile Gruppen

enthaltenden Substrat umsetzt.

29. Verwendung der oberflächenreichen Systeme gemäß den Ansprüchen 1 bis 4 und 13 bis 28 zur Fixierung von funktional und/oder morphologisch definierten, biologisch wirksamen Einheiten.

30. Verwendung der oberflächenreichen Systeme gemäß den Ansprüchen 1 bis 4 und 13 bis 29 zur Immobilisierung von Proteinen.

31. Verwendung der oberflächenreichen Systeme gemäß den Ansprüchen 1 bis 4 und 13 bis 30 zur Bindung von Enzymen.

32. Verwendung der oberflächenreichen Systeme gemäß den Ansprüchen 1 bis 4, 13 bis 30 zur Bindung von Blutproteinen und Blutfaktoren.

33. Verwendung der oberflächenreichen Systeme gemäß Anspruch 32, zur Bindung von Albumin, Immunoglobulinen, Faktoren der Blutgerinnung, Zellmembranproteinen und Peptidhormonen.

34. Verwendung der oberflächenreichen Systeme gemäß den Ansprüchen 1 bis 4, 13 bis 30, zur Immobilisierung von hochmolekularen, biogenen Substraten.

35. Verwendung der oberflächenreichen Systeme gemäß den Ansprüchen 1 bis 4, 13 bis 30 und 34, zur Immobilisierung von mit Farbstoffen kovalent markierten, hochmolekularen, biogenen Substraten.

36. Verwendung der oberflächenreichen Systeme gemäß den Ansprüchen 1 bis 35, als Katalysatorsysteme.

37. Verwendung der oberflächenreichen Systeme gemäß den Ansprüchen 1 bis 36, als Biokatalysatoren, insbesondere mit Enzymwirkung.

38. Verwendung der oberflächenreichen Systeme gemäß den Ansprüchen 1 bis 35, als bioaffine Indikatoren, beispielsweise als immobilisierte Substrate.

39. Verwendung der oberflächenreichen Systeme gemäß den Ansprüchen 1 bis 35, als bioaffine Sorbentien.

40. Verwendung der oberflächenreichen Systeme gemäß den Ansprüchen 1 bis 4 und 13 bis 30, zur Entfernung von nucleophile Gruppen enthaltenden Verunreinigungen aus wäßrigen Medien.

41. Verwendung der oberflächenreichen Systeme gemäß den Ansprüchen 1 bis 4, zur Umsetzung mit bi- oder multifunktionellen Substanzen, die mindestens eine nucleophile Gruppe zur Fixierung am Latex und mindestens eine weitere funktionelle Gruppe aufweisen, die zur spezifischen Wechselwirkung mit in wäßrigem Medium befindlichen Substanzen oder funktionellen Einheiten geeignet ist.

42. Verwendung der oberflächenreichen Systeme gemäß Anspruch 41, dadurch gekennzeichnet, daß die weitere funktionelle Gruppe eine komplexierende Wirkung ausübt.

43. Verwendung der oberflächenreichen Systeme gemäß den Ansprüchen 1 bis 4 und 42, als stationäre Phase in der präparativen organischen Chemie.

44. Verwendung der oberflächenreichen Systeme gemäß den Ansprüchen 1 bis 4 und 43 zur Peptidsynthese.

45. Verwendung der oberflächenreichen Systeme gemäß Anspruch 31 zur Bindung therapeutisch wirksamer Enzyme.

46. Verwendung der oberflächenreichen Systeme gemäß Anspruch 45 zur Bindung oral applizierbarer, therapeutisch wirksamer Enzyme.

47. Verwendung der oberflächenreichen Systeme gemäß den Ansprüchen 45 und 46 zur Bindung von Proteasen und/oder Lipasen und/oder Amylasen.

## Claims

1. Large surface area systems with reactive units for bonding substrates containing nucleophilic groups, characterised in that the reactive units for bonding the substrates containing the nucleophilic groups are, from the nature of the manufacture, part of a polymer latex prepared by emulsion polymerisation which is made up of latex particles measuring from 0.03 to 6 $\mu$m and the polymer latex itself is aggregated to form a large surface area system and/or is fixed to a carrier material with a large surface area.

2. Large surface area systems as claimed in claim 1, characterised in that the aggregation of the polymer latices to form a large surface area system is effected by spray drying below the minimum film forming temperature (MFT) of the individual polymer latex.

3. Large surface area systems as claimed in claim 1, characterised in that the aggregation of the polymer latices to form a large surface area system is effected by freeze-drying.

4. Large surface area systems as claimed in claim 1, characterised in that the aggregation of the polymer latices to form a large surface area system is effected by thermal coagulation, freezing or by precipitation with electrolytes or solvents.

5. Large surface area systems as claimed in claim 1, characterised in that the aggregation of the polymer latices to form a large surface area system is effected by precipitation with the substrate containing the nucleophilic group.

6. Large surface area systems as claimed in claims 1 and 5, characterised in that the substrate containing the nucleophilic groups is of the nature of a protein.

7. Large surface area systems as claimed in claims 1 and 5, characterised in that the substrates used

for precipitation and containing nucleophilic groups consist of functionally and/or morphologically defined, biologically active units.

8. Large surface area systems as claimed in claims 1, 5, 6 and 7, characterised in that the substrate containing the nucleophilic groups is an enzyme.

9. Large surface area systems as claimed in claims 1 and 8, characterised in that the substrate containing the nucleophilic groups consists of several distinguishable enzymes.

10. Large surface area systems as claimed in claims 1 and 7, characterised in that the substrates used for precipitation and containing nucleophilic groups consist of microorganisms or the sub-units thereof, viruses, eukaryotic or prokaryotic cells and/or the sub-units thereof or cell hybrids or cell organelles, such as mitochondria, microsomes, parts of membrane, cell nuclei or the sub-units thereof.

11. Large surface area systems as claimed in claim 1 and 2 to 4, characterised in that during aggregation of the polymer latices to form a structure with a large surface area, one or more types of substrates containing the nucleophilic groups are already present.

12. Large surface area systems as claimed in claim 1, characterised in that a latex dispersion is applied to a large surface area carrier material, the latex particles of this dispersion containing, as components, reactive groups for bonding the substrates containing the nucleophilic groups, and the latex is fixed to the carrier material.

13. Large surface area systems as claimed in claims 1 and 12, characterised in that the latex which is to be applied to the large surface area carrier material is film-forming and is fixed on the large surface area carrier material by simply drying it thereon.

14. Large surface area systems as claimed in claims 1 and 12, characterised in that the latex which is to be fixed is not film-forming *per se* and is fixed to the carrier material by the addition of small amounts of a film-forming latex or by means of the reactive units or other functional groups or reactive compounds.

15. Large surface area systems as claimed in claims 1 and 12, characterised in that the large surface area carrier material is composed essentially of organic material.

16. Large surface area systems as claimed in claim 15, characterised in that the organic material is made up of vinyl polymers, carbohydrates, proteins, polyamino acids, polyamides or polyesters.

17. Large surface area systems as claimed in claims 12, 15 and 16, characterised in that the large surface area carrier material is a non-woven mat of fibres.

18. Large surface area systems as claimed in claims 12, 15 and 16, characterised in that the large surface area carrier material is wadding.

19. Large surface area systems as claimed in claims 12, 15 and 16, characterised in that the large surface area carrier material is foamed.

20. Large surface area systems as claimed in claims 1 and 12, characterised in that the large surface area carrier material is made up of inorganic material.

21. Large surface area systems as claimed in claim 20, characterised in that the inorganic carrier material is in the form of a non-woven mat or in the form of a porous body.

22. Large surface area systems as claimed in claim 20, characterised in that the inorganic material consists of silicon dioxide or silicates, glass, aluminium oxide and/or other metal oxides, clays, sand, ceramics, carbon or stainless steel.

23. Large surface area systems as claimed in claims 1 to 22, characterised in that the polymer latex is synthesised from radically polymerisable monomers.

24. Large surface area systems as claimed in claims 1 and 23, characterised in that the polymer latex is based on acrylic acid and/or methacrylic acid derivatives and/or styrene and/or vinyl acetate.

25. Large surface area systems as claimed in claims 1 to 24, characterised in that the reactive units, as components of a polymer latex, are those which will react with the substrates containing nucleophilic groups in an aqueous solution and in a pH range of from 5-10, forming a covalent bond.

26. Large surface area systems as claimed in claim 25, characterised in that the reactive units are oxiran, keto, formyl, sulfonic acid halide, thioisocyanate groups, activated carboxylic acid esters and/or carboxylic acid anhydrides or activated double bonds.

27. Large surface area systems as claimed in claims 1 to 26, characterised in that they additionally contain a dye which is optionally pH- or redox-sensitive.

28. Use of the large surface area systems as claimed in claims 1 to 4 and 12, characterised in that the latices fixed to the carrier material are reacted with a substrate containing nucleophilic groups.

29. Use of the large surface area systems as claimed in claims 1 to 4 and 13 to 28, for fixing functionally and/or morphologically defined, biologically active units.

30. Use of the large surface area systems as claimed in claims 1 to 4 and 13 to 29 for immobilizing proteins.

31. Use of the large surface area systems as claimed in claims 1 to 4 and 13 to 30 for bonding enzymes.

32. Use of the large surface area systems as claimed in claims 1 to 4 and 13 to 30 for bonding blood proteins and blood factors.

33. Use of the large surface area systems as claimed in claim 32, for bonding albumin, immunoglobulins, blood coagulation factors, cell membrane proteins and peptide hormones.

34. Use of the large surface area systems as claimed in claims 1 to 4 and 13 to 30 for immobilising

high-molecular biogenic substrates.

35. Use of the large surface area systems as claimed in claims 1 to 4, 13 to 30 and 34, for immobilising high-molecular biogenic substrates covalently labelled with dyes.

36. Use of the large surface area systems as claimed in claims 1 to 35 as catalyst systems.

37. Use of the large surface area systems as claimed in claims 1 to 36 as biocatalysts, particularly with an enzyme activity.

38. Use of the large surface area systems as claimed in claims 1 to 35 as indicators with bioaffinity, for example as immobilised substrates.

39. Use of the large surface area systems as claimed in claims 1 to 35 as sorbents with bioaffinity.

40. Use of the large surface area systems as claimed in claims 1 to 4 and 13 to 30 for eliminating any impurities containing nucleophilic groups from aqueous media.

41. Use of the large surface area systems as claimed in claims 1 to 4 for reacting with bi- or multifunctional substances which have at least one nucleophilic group for fixing to the latex and at least one other functional group which is suitable for specifically interacting with substances or functional units found in an aqueous medium.

42. Use of the large surface area systems as claimed in claim 41 characterised in that the other functional group has a complexing activity.

43. Use of the large surface area systems as claimed in claims 1 to 4 and 42 as a stationary phase in preparative organic chemistry.

44. Use of the large surface area systems as claimed in claims 1 to 4 and 43 for peptide synthesis.

45. Use of the large surface area systems as claimed in claim 31 for bonding therapeutically active enzymes.

46. Use of the large surface area systems as claimed in claim 45 for bonding orally administrable, therapeutically active enzymes.

47. Use of the large surface area systems as claimed in claims 45 and 46 for bonding proteases and/or lipases and/or amylases.

**Revendications**

1. Systèmes à grande surface comportant des unités réactives pour la fixation de substrats contenant des groupes nucléophiles, caractérisés en ce que les unités réactives pour la fixation des substrats contenant des groupes nucléophiles font partie, par préparation, d'un latex polymère préparé par polymérisation en émulsion, qui est formé de particules de latex de 0,03 à 6 μm de grosseur et en ce que le latex polymère est lui-même agrégé en un système à grande surface et/ou est fixé à un matériau de support à grande surface.

2. Systèmes à grande surface selon la revendication 1, caractérisés en ce que l'agrégation des latex polymères en un système à grande surface est effectuée par séchage par pulvérisation au-dessous de la température minimale de formation de films du latex polymère individuel.

3. Systèmes à grande surface selon la revendication 1, caractérisés en ce que l'agrégation des latex polymères en un système à grande surface est effectuée par lyophilisation.

4. Systèmes à grande surface selon la revendication 1, caractérisés en ce que l'agrégation des latex polymères en un système à grande surface est effectuée par coagulation thermique, par congélation ou par précipitation avec des électrolytes ou des solvants.

5. Systèmes à grande surface selon la revendication 1, caractérisés en ce que l'agrégation des latex polymères en un système à grande surface est effectuée par précipitation avec le substrat contenant les groupes nucléophiles.

6. Systèmes à grande surface selon la revendication 1 ou 5, caractérisés en ce que le substrat contenant les groupes nucléophiles a le caractère d'une protéine.

7. Systèmes à grande surface selon la revendication 1 ou 5, caractérisés en ce que les substrats contenant des groupes nucléophiles qui sont utilisés pour la précipitation sont constitués par des unités à activité biologique, définies fonctionnellement et/ou morphologiquement.

8. Systèmes à grande surface selon l'une quelconque des revendications 1 et 5 à 7, caractérisés en ce que le substrat contenant les groupes nucléophiles est un enzyme.

9. Systèmes à grande surface selon la revendication 1 ou 8, caractérisés en ce que le substrat contenant les groupes nucléophiles se compose de plusieurs enzymes différents.

10. Systèmes à grande surface selon la revendication 1 ou 7, caractérisés en ce que les substrats contenant des groupes nucléophiles, utilisés pour la précipitation sont constitués par des micro-organismes ou leurs sous-unités, des virus, des cellules eucaryotiques ou procaryotiques et/ou leurs sous-unités ou hybrides cellulaires ou des organelles cellulaires tels que mitochondries, microsomes, parties de membrane, noyaux cellulaires ou leurs sous-unités.

11. Systèmes à grande surface selon l'une quelconque des revendications 1 et 2 à 4, caractérisés en ce qu'une ou plusieurs sortes des substrats contenant les groupes nucléophiles sont déjà présents lors de l'agrégation des latex polymères en un produit à grande surface.

12. Systèmes à grande surface selon la revendication 1, caractérisés en ce qu'il est appliqué, sur un

matériau de support à grande surface, une dispersion de latex dont les particules de latex comportent, comme constituants, des groupes réactifs pour la fixation des substrats contenant les groupes nucléophiles, puis le latex est fixé au matériau de support.

13. Systèmes à grande surface selon la revendication 1 ou 12, caractérisés en ce que le latex à appliquer sur le matériau de support à grande surface est filmogène et est fixé par simple séchage sur le matériau de support à grande surface.

14. Systèmes à grande surface selon la revendication 1 ou 12, caractérisés en ce que le latex à fixer n'est pas filmogène en soi et est fixé au matériau de support par adjonction de faibles quantités d'un latex filmogène ou bien au moyen des unités réactives ou d'autres groupes fonctionnels ou composés réactifs.

15. Systèmes à grande surface selon la revendication 1 ou 12, caractérisés en ce que le matériau de support à grande surface est composé essentiellement de matière organique.

16. Systèmes à grande surface selon la revendication 15, caractérisés en ce que la matière organique est faite de polymères vinyliques, de glucides, de protéines, de polyaminoacides, de polyamides ou de polyesters.

17. Systèmes à grande surface selon l'une quelconque des revendications 12, 15 et 16, caractérisés en ce que le matériau de support à grande surface se présente sous forme de nappe de fibres.

18. Systèmes à grande surface selon l'une quelconque des revendications 12, 15 et 16, caractérisés en ce que le matériau de support à grande surface se présente sous la forme d'une ouate.

19. Systèmes à grande surface selon l'une quelconque des revendications 12, 15 et 16, caractérisés en ce que le matériau de support à grande surface est expansé.

20. Systèmes à grande surface selon la revendication 1 ou 12, caractérisés en ce que le matériau de support à grande surface est composé de matière inorganique.

21. Systèmes à grande surface selon la revendication 20, caractérisés en ce que le matériau de support inorganique se présente sous forme de nappe ou sous forme d'un corps poreux.

22. Systèmes à grande surface selon la revendication 20, caractérisés en ce que la matière inorganique est faite de bioxyde de silicium ou de silicates, de verre, d'oxyde d'aluminium et/ou d'autres oxydes métalliques, d'argiles, de sable, de céramique, de charbon ou d'acier inoxydable.

23. Systèmes à grande surface selon l'une quelconque des revendications 1 à 22, caractérisés en ce que le latex polymère est fait de monomères susceptibles de polymérisation radicalaire.

24. Systèmes à grande surface selon la revendication 1 ou 23, caractérisés en ce que le latex de polymère est formé à base de dérivés d'acide acrylique et/ou méthacrylique et/ou à base de styrène et/ou à base d'acétate de vinyle.

25. Systèmes à grande surface selon l'une quelconque des revendications 1 à 24, caractérisés en ce que les unités réactives sont, en tant que constituants d'un latex polymère, des unités qui réagissent avec les substrats contenant des groupes nucléophiles en solution aqueuse et dans la gamme de pH comprise entre 5 et 10 en formant une liaison covalente.

26. Systèmes à grande surface selon la revendication 25, caractérisés en ce que les unités réactives sont des groupements oxiranne, céto, formyle, halogénure d'acide sulfonique, thioisocyanate, des esters d'acides carboxyliques activés et/ou des anhydrides d'acides carboxyliques ou des doubles liaisons activées.

27. Systèmes à grande surface selon l'une quelconque des revendications 1 à 26, caractérisés en ce qu'ils contiennent en plus un colorant éventuellement sensible au pH ou à l'oxydo-réduction.

28. Utilisation des systèmes à grande surface selon l'une quelconque des revendications 1 à 4 et 12, caractérisée en ce qu'on fait réagir les latex fixés sur le matériau de support avec un substrat contenant des groupes nucléophiles.

29. Utilisation des systèmes à grande surface selon l'une quelconque des revendications 1 à 4 et 13 à 28 pour la fixation d'unités à activité biologique, définies fonctionnellement et/ou morphologiquement.

30. Utilisation des systèmes à grande surface selon l'une quelconque des revendications 1 à 4 et 13 à 29 pour l'immobilisation de protéines.

31. Utilisation des systèmes à grande surface selon l'une quelconque des revendications 1 à 4 et 13 à 30 pour la fixation d'enzymes.

32. Utilisation des systèmes à grande surface selon l'une quelconque des revendications 1 à 4 et 13 à 30 pour la fixation de protéines sanguines et de facteurs sanguins.

33. Utilisation des systèmes à grande surface selon la revendication 32 pour la fixation d'albumine, d'immunoglobulines, de facteurs de la coagulation sanguine, de protéines de la membrane cellulaire et d'hormones peptidiques.

34. Utilisation des systèmes à grande surface selon l'une quelconque des revendications 1 à 4 et 13 à 30 pour l'immobilisation de substrats biogènes de poids moléculaire élevé.

35. Utilisation des systèmes à grande surface selon l'une quelconque des revendications 1 à 4, 13 à 30 et 34 pour l'immobilisation de substrats biogènes de poids moléculaire élevé, marqués en covalence avec des colorants.

36. Utilisation des systèmes à grande surface selon l'une quelconque des revendications 1 à 35 comme systèmes catalyseurs.

37. Utilisation des systèmes à grande surface selon l'une quelconque des revendications 1 à 36 comme biocatalyseurs, en particulier à activité enzymatique.

38. Utilisation des systèmes à grande surface selon l'une quelconque des revendications 1 à 35 comme indicateurs bio-affines, par exemple comme substrats immobilisés.

39. Utilisation des systèmes à grande surface selon l'une quelconque des revendications 1 à 35 comme sorbants bio-affines.

40. Utilisation des systèmes à grande surface selon l'une quelconque des revendications 1 à 4 et 13 à 30 pour l'élimination d'impuretés contenant des groupes nucléophiles de milieux aqueux.

41. Utilisation des systèmes à grande surface selon l'une quelconque des revendications 1 à 4 pour leur mise en réaction avec des substances bi- ou multifonctionnelles, qui présentent au moins un groupe nucléophile pour leur fixation au latex et au moins un autre groupe fonctionnel, qui convient pour l'interaction spécifique aves des substances se trouvant en milieu aqueux ou des unités fonctionnelles.

42. Utilisation des systèmes à grande surface selon la revendication 41, caractérisée en ce que l'autre groupe fonctionnel exerce un effet complexant.

43. Utilisation des systèmes à grande surface selon l'une quelconque des revendications 1 à 4 et 42 comme phase stationnaire en chimie organique expérimentale.

44. Utilisation des systèmes à grande surface selon l'une quelconque des revendications 1 à 4 et 43 pour la synthèse de peptides.

45. Utilisation des systèmes à grande surface selon la revendication 31 pour la fixation d'enzymes à activité thérapeutique.

46. Utilisation des systèmes à grande surface selon la revendication 45 pour la fixation d'enzymes à activité thérapeutique, administrables par voie orale.

47. Utilisation des systèmes à grande surface selon la revendication 45 ou 46 pour la fixation de protéases et/ou de lipases et/ou d'amylases.